# NEW EUROPEAN PATENT SPECIFICATION

(11) **EP 3 728 550 B2**
(45) Date of publication and mention of the opposition decision: **25.02.2026**
(45) Mention of the grant of the patent: 17.02.2021
(21) Application number: 19756029.5
(22) Date of filing: 14.08.2019
(51) Int. Cl.: C12M 1/00

(54) **LID CONFIGURATION FOR A BIOREACTOR**
DECKELKONFIGURATION FÜR EINEN BIOREAKTOR
CONFIGURATION DE COUVERCLE POUR UN BIORÉACTEUR

(30) Priority: 15.08.2018 NL 2021470
(43) Date of publication of application: 28.10.2020
(73) Proprietor: APPLIKON BIOTECHNOLOGY B.V., 2629 JG Delft (NL)
(72) Inventor: WALVOORT, Wilbert Timotheüs, 2629 JG DELFT (NL); OOSTRA, Jaap, 2629 JG DELFT (NL)
(74) Representative: Algemeen Octrooi- en Merkenbureau B.V.
(86) International application number: PCT/NL2019/050530
(87) International publication number: WO 2020/036489

(56) References cited:
- WO-A1-2017/083705
- WO-A1-2017/083705
- JP-B2- 5 545 689
- US-A1- 2016 053 213
- US-A1- 2016 053 213
- US-A1- 2016 281 043
- US-A1- 2017 191 017
- US-A1- 2017 349 874
- US-A1- 2017 369 828

## Description

### FIELD OF THE INVENTION

The present invention relates to a lid configuration for a bioreactor, comprising a lid configured for connection with a vessel of the bioreactor, the vessel during use containing a bioreactor fluid, a bioreactor comprising such a lid configuration, a bioreactor configuration kit comprising such a lid configuration, as well as a method for producing such a lid configuration.

### BACKGROUND OF THE INVENTION

A bioreactor generally relates to a manufactured or engineered device or system that supports a biologically active environment. Such a bioreactor, e.g. as disclosed in the prior art documents WO 2017/083705 A1, US 2017/349874 A1 and US 2017/369828 A1, usually comprises a vessel in which a biological process is carried out which involves organisms or biochemically active substances derived from such organisms. This process can either be aerobic or anaerobic. Such bioreactors are commonly cylindrical, ranging in size from milliliters to cubic meters, and are often made of glass and/or stainless steel. A bioreactor may also refer to a device or system designed to grow cells or tissues in the context of cell culture. Such devices are being developed for use in tissue engineering or biochemical engineering. Based on their mode of operation, a bioreactor may be classified as batch, fed batch or continuous.

Organisms growing in bioreactors may be submerged in liquid medium or may be attached to the surface of a solid medium. Submerged cultures may be suspended or immobilized. Suspension bioreactors can use a wider variety of organisms, since special attachment surfaces are not needed, and can operate at much larger scale than immobilized cultures, due to optimal utilization of cell membrane surface area. In a continuously operated process the organisms will remain in the reactor and byproducts will be removed from the reactor with the effluent.

A typical bioreactor comprises the following parts: an agitator or impeller - used for the mixing of the contents of the reactor which keeps the "cells" in the perfect homogenous condition for better transport of nutrients and oxygen to the cells, a baffle - used to break the vortex formation in the vessel, which is usually highly undesirable as it changes the centre of gravity of the system and consumes additional power without adding to the mixing, a sparger - in aerobic cultivation process, the purpose of the sparger is to supply adequate gasses (such as oxygen or CO₂) to the growing cells, and a jacket - the jacket provides the annular area for circulation of certain temperature of water which keeps the temperature of the bioreactor at a desired value, although the use of Peltier heating or heating blankets is also conceivable. Bioreactors generally are classified as having a volume of more than 20 liters or a volume of less than 20 liters.

US 2017/0321178 A discloses a design, a method of fabrication, and applications of a three-dimensional (3D) bioreactor for cell expansion and cell secreted substance production. The bioreactor is composed of non-random interconnected voids providing a continuous three-dimensional surface area for cell adherence and growth.

US 2017/0211026 A furthermore discloses a membrane, including a polymeric network configured to separate a first fluid and a second fluid, where the first and second fluids are different; and a plurality of enzymatic reactive components embedded within the polymeric network. The bioreactor may include a lattice of three dimensional structures, each structure including a membrane having: a polymeric network configured to separate a first fluid and a second fluid, where the first and second fluids are different; and a plurality of enzymatic reactive components embedded within the polymeric network.

US 2017/191017 A1 discloses a lid to be connected to disposable bioreactors and provided with several ports.

In practice, bioreactors are relatively complex and expensive to produce (in particular bioreactors having a volume of less than 20 liters), especially in low quantities, and even more difficult and expensive to configure by an end user, for instance a biologist. Such a biologist knows the parameters needed for the respective bioreactor process to be properly used or studied, such as the production of insulin, and the type of bioreactor design/configuration required to achieve this.

The abovementioned problem of complex and expensive production has been partially addressed by some manufacturers by providing a bioreactor lid and/or a reactor vessel made of plastic. The relatively inexpensive and easy to produce plastic lid and/or bioreactor vessel can be disposed of after use. Some manufacturers advantageously use 3D-printing to produce the plastic lid (and/or bioreactor vessel).

Such a single-use or disposable solution has several further advantages, apart from reducing complexity and production cost. Single-use technologies also reduce assembly/disassembly, cleaning, sterilization and calibration demands. Some estimates show cost savings of more than 60% with single use systems compared to fixed asset stainless steel bioreactors. In pharmaceutical production, complex qualification and validation procedures can be made easier, and will finally lead to significant cost reductions. The application of single-use bioreactors reduces the risk of cross-contamination and enhances the biological and process safety. Single-use applications are suitable for any kind of biopharmaceutical product. Single-use bioreactors also contain fewer parts compared with conventional bioreactors, so the initial and maintenance costs are reduced.

However, a problem with disposable or single-use bioreactors is that the sensor or controllers elements to be used, i.e. to sense/measure or control bioreactor process parameters, such as temperature, pH, pressure, oxygen content, et cetera, are often still made of expensive or complex materials, such as stainless steel. Therefore, in practice, the above parts still have to be cleaned, disinfected, assembled/disassembled, et cetera, after use, negating the benefits of having a single-use or disposable lid and/or vessel. Moreover, such disposable or single-use bioreactors are difficult to configure or customize by the end user, due to the relatively limited range of available sensor or controller elements, i.e. the end user is too limited in customizing the bioreactor according to his wishes.

### OBJECT OF THE INVENTION

An object of the invention is therefore to provide a lid configuration for a bioreactor, wherein the lid configuration, i.e. with the sensor or controller elements, is substantially disposable.

Another object of the invention is to provide a lid configuration for a bioreactor, wherein the end user is provided with a higher degree of configurability and customizability than with present-day lid configurations.

### DESCRIPTION OF THE INVENTION

Hereto, the lid configuration for a bioreactor according to the invention (e.g. the lid or head plate), is provided with one or more 3D-printed controller elements for, during use, controlling one or more parameters of the bioreactor, in particular the bioreactor fluid. The lid configuration is characterized in that the vessel has a volume of less than 3 liters, wherein the one or more 3D-printed controller elements comprise one or more 3D-printed inlets or outlets comprising 3D-printed coaxial inlet and/or outlet tubes extending into the vessel. In other words, the lid configuration for a bioreactor is characterized by the lid being provided with one or more 3D-printed inlets or outlets comprising 3D-printed coaxial inlet and/or outlet tubes extending into the vessel.

The Applicant has found that the aforementioned lid configuration allows the lid configuration, i.e. with the controller elements, to be substantially disposable (i.e. as a whole).

At the same time, the end user is provided with a much higher degree of configurability and customizability than with present-day lid configurations, due to one or more of the controller elements being 3D-printed.

For example, the inlets or outlets may be spargers, water inlets, gas outlets, nutrient inlets, anti-foam inlets, steam inlets, inoculant inlets, et cetera. Therein, both "fluids", such as gasses or liquids, as well as solid materials may be transported through the inlets or outlets. The Applicant has found that in particular the bioreactor components that are continuously or frequently exposed to a gas or fluid can advantageously be made "single-use", produced with 3D-printing, due to these components not having to be cleaned afterwards.

The fluid in the vessel of the bioreactor may e.g. be a liquid, with a layer of foam on top of said liquid. A further advantage of 3D-printed inlets or outlets, especially when the inlet has a tube that extends into the vessel of a bioreactor, is that fluids may be injected directly into the liquid (instead of being dripped onto the foam). This results in a better controllable and more precise biological process inside the bioreactor.

A further advantage of 3D-printed inlets or outlets is that a shape of the inlet or outlet may be optimally tailored to a specific function. Using 3D printing techniques, shapes and designs become obtainable which were previously impossible to manufacture. As a mere example, it becomes possible to match the inlet end of an outlet to the shape of the bottom of the vessel, to allow (almost) every drop of fluid to be sucked from the bottom of the vessel, with hardly any spoiling. As a further example, an inlet end of an outlet may have one or more openings in its circumferential wall, e.g. only at the top portion (the portion facing the lid) or only at the bottom portion (the portion facing a bottom of the vessel). This allows to suck only very specific types of fluid from the vessel. For example this allows to suck supernatant liquids, i.e. fluids which float on top of other fluids just like oil floats on top of water, from the vessel.

A further advantage of 3D-printed inlets or outlets is that the coaxial inlet and/or outlet tubes may be manufactured relatively cheaply. Especially when the vessel is relatively small, i.e. less than 3 liters, and when relatively many components are needed, e.g. an impeller, one or more sensors such as a temperature sensor, a fluid level sensor, a pH sensor, and several inlets and/or outlets, e.g. more than 3, such as 4, 5, 6 or more inlets and/or outlets, it may become problematic to sufficiently miniaturize all components and they may not fit inside the vessel.

It should be understood that in the below, if reference is made to 'controller elements', this may refer to inlets, outlets, or other controller elements (such as an agitation element).

It should be understood that, within the context of this patent application, 3D-printing is any of various processes in which material is joined or solidified to create a three-dimensional object, with material being added together (such as liquid molecules or powder grains being fused together). 3D-printing is often used in both rapid prototyping and additive manufacturing (AM). Objects can be of almost any shape or geometry and typically are produced using digital model data from a 3D model or another electronic data source such as an Additive Manufacturing File (AMF) file (usually in sequential layers). There are many different technologies, like stereo-lithography (SLA) or fused deposit modeling (FDM). Thus, unlike material removed from a stock in the conventional machining process, 3D printing or AM builds a three-dimensional object from computer-aided design (CAD) model or AMF file, usually by successively adding material layer by layer.

The term "3D-printing" originally relates to a process that deposits a binder material onto a powder bed with inkjet printer heads layer by layer. More recently, the term is being used in popular vernacular to encompass a wider variety of additive manufacturing techniques. Therefore, in the context of this patent application, the term "additive manufacturing techniques" can be used interchangeably with the term "3D-printing".

The Applicant foresees particular use with bioreactors in which many different biological processes and different cell types and therapies for addressing diseases can be used or created, including diabetes mellitus types 1 and 2, i.e. "regenerative medicine" bioreactors, or for the production of insulin. In addition the Applicant foresees advantageous use with "tissue engineering bioreactors".

An embodiment relates to an aforementioned lid configuration, wherein the one or more 3D-printed controller elements further comprise a 3D-printed agitation element. In particular when agitation elements with pitched-blade impellers are used, which are flat and set at an angle with respect to the plane perpendicular to the shaft, such as a 45° angle, to produce a simultaneous axial and radial flow, 3D-printing provides many advantages. By 3D-printing the agitation element, the Applicant found that the end user can define highly complex shapes to achieve optimal mixing performance, creating e.g. a higher oxygen mass transfer rate and less shear force on the cells.

An embodiment thus relates to an aforementioned lid configuration, wherein the agitation element comprises an impeller, such as a (3D-printed) screw or helix.

An embodiment thus also relates to an aforementioned lid configuration, wherein the agitation element comprises a 3D-printed shaft. The drive motor connection for the agitation element, usually positioned above the lid, however, is preferably made in a conventional way - i.e. generally not having 3D-printed parts (in view of wear and tear caused by rotational movement).

An embodiment relates to an aforementioned lid configuration, wherein the 3D-printed controller elements and/or the 3D-printed lid are made of a biodegradable material, such as a biodegradable plastic. Biodegradable means that the material used should show evidence of breaking down in nature until microorganisms digest the material and the material returns to nature. This process must happen in a reasonably short period of time after disposal. When something is just degradable, it means that it will break down into smaller pieces and will not necessarily be digested by microorganisms. Preferably, the material will be completely biodegrade fast enough in a certain environment, i.e. the material then constitutes a compostable plastic. The material breaks down into carbon dioxide, water, and biomass at the same rate as paper and fully disintegrates in a compost pile, wherein no toxic residues are left and the compost supports plant growth.

Preferably, the biodegradable material comprises ABS (acrylonitrile butadiene styrene) and PLA (polylactic acid). ABS is a thermoplastic that is advantageous for 3D printing because of its strength and durability. PLA is often compostable, though it requires a very specific temperature and environment to do so. It is made from products like corn starch, sugar cane, and tapioca root so it can be absorbed by microorganisms.

An embodiment relates to an aforementioned lid configuration, wherein the 3D-printed controller elements and/or the lid are made of a biocompatible material. The Applicant foresees particular use of bioreactors with "bedside medicine". Therein, it is important that the 3D-printed controller elements and/or the lid are made of a biocompatible material, wherein product contact surfaces are "not reactive, additive or absorptive", i.e. the presence of leachables and extractables is minimized. Extractables are defined as container-closure contaminants that can be extracted from plastic materials under 'forcing' conditions, i.e. high temperatures and pressures, organic solvents, et cetera. In contrast, leachables are defined as those contaminants that can be extracted from the container closures under standard ICH storage conditions. As such, leachables are a sub-set of extractables.

A thorough understanding and control of extractables and leachables in liquid and semi-solid products has long been a regulatory requirement - for instance laid down in regulatory guidelines such as EudraLex, i.e. the collection of rules and regulations governing medicinal products in the European Union (in particular in Volume 4, Chapter 5, which relates to Good Manufacturing Practices or "GMP"). The use of biocompatible materials is therefore deemed highly advantageous by the Applicant.

An embodiment relates to an aforementioned lid configuration, wherein the 3D-printed controller elements and/or the 3D-printed lid are produced by means of laser sintering. Laser sintering is particularly useful for use with limited-run manufacturing to produce end-use parts, such as configurable bioreactors, in particular for use in laboratory settings.

An embodiment thus relates to an aforementioned lid configuration, wherein the lid is a 3D-printed lid.

Another aspect of the invention relates to a bioreactor.

The use of 3D-printing for producing the sensor or controller elements is particularly useful for use with relatively low volume, configurable bioreactors, for instance for use with small scale experiments.

An embodiment relates to an aforementioned bioreactor, wherein the vessel is made of plastic, preferably biodegradable plastic, such that the bioreactor as a whole can be disposed of after the process or experiment is complete.

An embodiment relates to an aforementioned bioreactor, wherein the vessel is a 3D-printed vessel to further optimize configurability and customizability.

An embodiment relates to an aforementioned bioreactor, wherein the vessel of the bioreactor comprises a 3D-printed outlet, e.g. a side outlet, preferably arranged in the bottom half of the vessel, more preferably arranged in the bottom quarter of the vessel, such as in the bottom 10% of the vessel. An advantage of such a 3D-printed side outlet is that it allows a customization of the connection port, associated with the side outlet such that any device can be connected to the 3D-printed outlet, and that the bioreactor can thus be optimally tailored to the intended use of the bioreactor.

Yet another aspect of the invention relates to a bioreactor configuration kit according to claim 12.

Thus, a wide range of 3D-printed controller elements, to be connected to the lid, can be produced in advance. When the bioreactor is to be used or to become operational, the 3D-printed controller elements are then selected from the range of 3D-printed controller elements by the end user, according to his or her wishes or requirements.

Another aspect of the invention relates to the use of a bioreactor or bioreactor kit as described in claim 13.

Another aspect of the invention relates to a method according to claim 14.

An embodiment relates to an aforementioned method, further comprising the steps of:
- determining the process or experiment to be carried out with the bioreactor,
- determining the 3D-printed controller elements, in particular the (type of) inlets or outlets, required for carrying out the experiment or process, and
- 3D-printing only those controller elements required for carrying out the experiment of process.

Thus, a highly individual, specific bioreactor can be designed from the start, e.g. for use with a unique experiment or process.

Preferably, prior to determining the 3D-printed controller elements required for carrying out the experiment or process, a design, such as a 3D digital design, is made of the bioreactor for carrying out the process or experiment, and the 3D-printed controller elements required for carrying out the experiment or process are determined from the design, such as a 3D digital design comprised by an STL file.

An embodiment thus relates to an aforementioned method, further comprising the steps of:
- determining the process or experiment to be carried out with the bioreactor,
- determining the 3D-printed controller elements required for carrying out the experiment or process, and
- selecting the controller elements required for carrying out the experiment of process from the aforementioned bioreactor configuration kit.

### BRIEF DESCRIPTION OF THE DRAWINGS

The present invention will be explained hereafter with reference to exemplary embodiments of the lid configuration and bioreactor according to the invention and with reference to the drawings. Therein:
Figure 1 shows a perspective view of an exemplary embodiment of a bioreactor with a lid configuration according to the invention;
Figure 2 shows a perspective view of the exemplary embodiment of the bioreactor according to figure 1, but now from a different angle;
Figure 3 shows a schematic top view of the exemplary embodiment of the lid configuration according to figures 1 and 2;
Figure 4 shows a perspective view of a further exemplary embodiment of a bioreactor lid;
Figure 5 shows a detailed view of the bioreactor lid of Figure 4; and
Figure 6 shows a view from below of the bioreactor lid of Figures 4 and 5.

### DETAILED DESCRIPTION

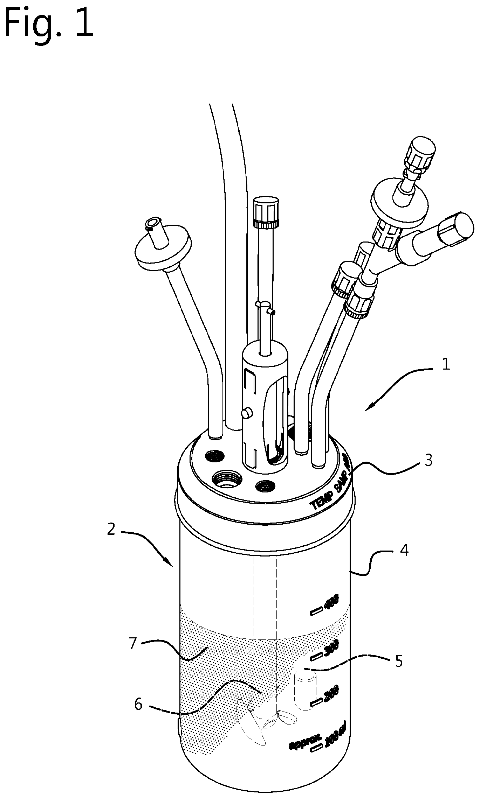

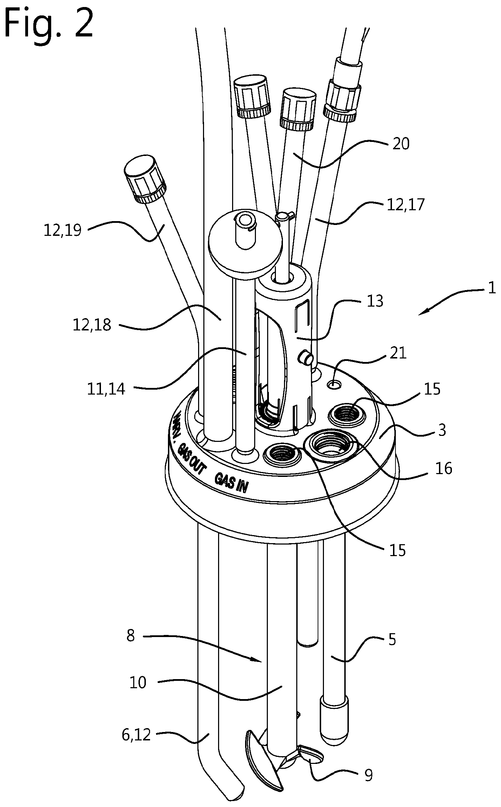

Figures 1-3 will be discussed in conjunction. Figure 1 shows a bioreactor 2 with a lid configuration/assembly 1, comprising a preferably 3D-printed lid 3 configured for connection with a vessel 4 of the bioreactor 2. The lid 3 may also be produced by e.g. injection molding, although 3D-printing the lid 3 increases the configurability or customizability of the lid configuration 1 and bioreactor 2. The bioreactor 2 preferably concerns a "regenerative medicine" bioreactor 2, such as for the production of insulin, or a "tissue engineering bioreactor". The vessel 4 during use contains a bioreactor fluid 7. The vessel 4 is preferably made of plastic. The vessel 4 may have a height of, for instance, 20 - 40 cm and/or a diameter of 10 - 30 cm, although other dimensions are also conceivable. The 3D-printed lid 3 is provided with one or more 3D-printed sensor 5 or controller 6 elements for, during use, sensing or controlling one or more parameters, such as process conditions, of the bioreactor 2, in particular the bioreactor fluid 7. In the exemplary embodiment as shown, the one or more 3D-printed sensor 5 or controller 6 elements comprise a 3D-printed agitation element 8, with a 3D-printed impeller 9 and a 3D-printed shaft 10. The agitation element 8 is driven by a drive motor (not shown) via a drive motor connection 13 arranged above the lid 3. Preferably, the agitation element 8 concerns a screw 9, such as a pitched-blade impeller 9, the blades of which are flat and set at an angle with respect to the plane perpendicular to the shaft 10, such as a 45° angle, to produce a simultaneous axial and radial flow.

The one or more 3D-printed sensor 5 or controller 6 elements may comprise one or more 3D-printed inlets 11 or outlets 12, such as a fluid inlet 11 in the form of a gas inlet 14. The outlet 12 may comprise a fluid outlet 12 in the form of a sample outlet 17, a gas outlet 18 or a harvest outlet 19. The inlets 11 and outlets 12 could be used not just for transporting gasses or liquids, but also for transporting solid materials or substances. The sensor 5 or controller elements 6 may further comprise one or more miscellaneous inlet/outlets 20. The lid 3 may also be provided with ports 15 for level sensors (not shown), a port 16 for a pH sensor (not shown), or a port 21 for a temperature sensor 5. The ports 15, 16, 21 could, of course, also receive other types of sensor elements.

Preferably, the 3D-printed sensor 5 or controller 6 elements and/or the 3D-printed lid 3 are made of a biodegradable material, such as a biodegradable plastic. More preferably, the 3D-printed sensor 5 or controller 6 elements and/or the 3D-printed lid 3 are made of a biocompatible material. The 3D-printed sensor 5 or controller elements 6 and/or the 3D-printed lid 3 can be produced by means of laser sintering, which is particularly useful for use with limited-run manufacturing to produce end-use parts, such as configurable bioreactors 2, in particular for use in laboratory settings.

The lid configuration 1 could also be part of a bioreactor configuration kit (not shown), i.e. a kit of parts, comprising:
- a vessel 4, the vessel 4 during use containing a bioreactor fluid 7,
- a preferably 3D-printed lid 3 configured for connection with the vessel 4 of the bioreactor 2,
- one or more 3D-printed sensor 5 or controller 6 elements, to be connected to the 3D-printed lid 3, for, during use, sensing or controlling one or more parameters of the bioreactor 2, in particular the bioreactor fluid 7.

A method for producing the lid configuration 1 may comprise the steps of:
- providing the lid 3, such as by 3D-printing, configured for connection with the vessel 4 of the bioreactor 2, the vessel 4 during use containing a bioreactor fluid 7,
- 3D-printing the one or more sensor 5 or controller 6 elements for, during use, sensing or controlling one or more parameters of the bioreactor 2, in particular the bioreactor fluid 7, and
- connecting the one or more sensor 5 or controller 6 elements to the lid 3.

The method may further comprise the steps of:
- determining the process or experiment to be carried out with the bioreactor 2,
- determining the 3D-printed sensor 5 or controller 6 elements required for carrying out the experiment or process, and
- 3D-printing only those sensor 5 or controller 6 elements required for carrying out the experiment of process.

Preferably, prior to determining the 3D-printed sensor 5 or controller 6 elements required for carrying out the experiment or process, a design, such as a 3D digital design, is made of the bioreactor 2 for carrying out the process or experiment, and the 3D-printed sensor 5 or controller 6 elements required for carrying out the experiment or process are determined from the design.

The method may further comprise the steps of:
- determining the process or experiment to be carried out with the bioreactor 2,
- determining the 3D-printed sensor 5 or controller 6 elements required for carrying out the experiment or process, and
- selecting the sensor 5 or controller 6 elements required for carrying out the experiment of process from the bioreactor configuration kit.

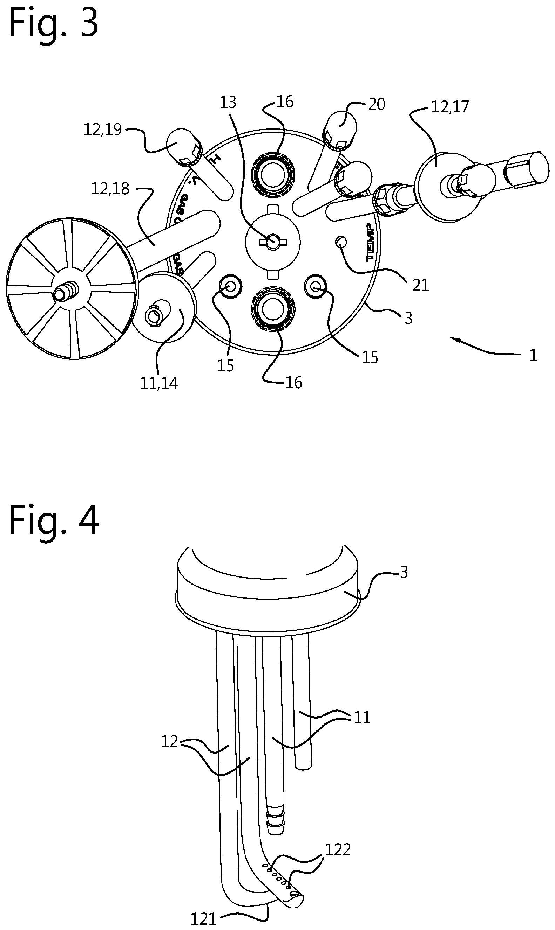

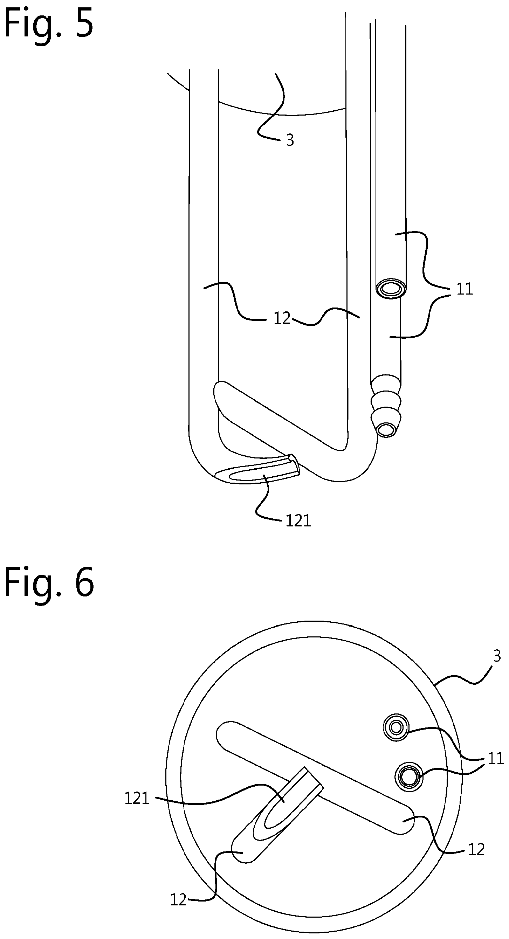

A further bioreactor lid is shown with respect to Figures 4 - 6, which will be described in conjunction. Shown here is a lid 3 of a bioreactor. The lid 3 is provided with four controller elements: two inlets 11 and two outlets 12. The outlets 12 are made with a 3D printing technique, which allows the outlets 12 to have a very specific shape, as may be desired. In the shown examples, mainly visible in Figures 5 and 6, a first inlet end 121 of an outlet 12 has a curved portion that faces a bottom of the bioreactor (that faces away from the lid 3). The inlet end 121 is here designed and shaped to match with said bottom of the vessel, allowing every drop of bioreactor fluid to be sucked from the vessel.

Also inlet end 122 of outlet 12 has a shape that is designed and shaped to its specific needs and that may be costly or impossible to obtain when made with production techniques other than 3D printing. The inlet end 122 of outlet 12, mainly visible in Figure 4, has a number of openings in the upper side of the circumferential wall thereof (in the side that faces the lid 3). By adapting the penetration depth of outlet 12 inside the vessel, supernatant fluid may be sucked from the vessel with such an outlet 12 having inlet end 122.

Also inlets 11 have an inlet tube that extends into the vessel of the bioreactor, allowing fluids (liquids or gasses) to be added directly into the bioreactor fluid (instead of being dripped onto a layer of foam that rests onto the bioreactor fluid). Also the inlets 11 are 3D printed.

### LIST OF REFERENCE NUMERALS

- 1.: Lid configuration
- 2.: Bioreactor
- 3.: Lid
- 4.: Bioreactor vessel
- 5.: Sensor elements
- 6.: Controller elements
- 7.: Bioreactor fluid
- 8.: Agitation element
- 9.: Impeller
- 10.: Shaft
- 11.: Fluid inlet
- 12.: Fluid outlet
- 13.: Drive motor connection
- 14.: Gas inlet
- 15.: Port for level sensor
- 16.: Port for pH sensor
- 17.: Sample outlet
- 18.: Gas outlet
- 19.: Harvest outlet
- 20.: Miscellaneous inlet/outlet
- 21.: Temperature sensor port
- 121.: Inlet end of outlet
- 122.: Inlet end of outlet

## Claims

1. Lid configuration (1) for a bioreactor (2), comprising:
- a lid (3) configured for connection with a vessel (4) of the bioreactor (2), the vessel (4) during use containing a bioreactor fluid (7), the lid (3) being provided with one or more 3D-printed controller (6) elements for, during use, controlling one or more parameters of the bioreactor (2), in particular the bioreactor fluid (7), **characterized, in that** the vessel has a volume of less than 3 liters, wherein the one or more 3D-printed controller (6) elements comprise one or more 3D-printed inlets (11) or outlets (12) comprising 3D-printed coaxial inlet (11) and/or outlet (12) tubes extending into the vessel.

2. Lid configuration (1) according to claim 1, wherein the one or more 3D-printed controller (6) elements comprise a 3D-printed agitation element (8).

3. Lid configuration (1) according to claim 2, wherein the agitation element (8) comprises a 3D-printed impeller, such as a screw (9).

4. Lid configuration (1) according to claim 2 or 3, wherein the agitation element (8) comprises a 3D-printed shaft (10).

5. Lid configuration (1) according to any one of the preceding claims, wherein the 3D-printed controller (6) elements and/or the lid (3) are made of a biodegradable material, such as a biodegradable plastic.

6. Lid configuration (1) according to any one of the preceding claims, wherein the 3D-printed controller (6) elements and/or the lid (3) are made of a biocompatible material.

7. Lid configuration (1) according to any one of the preceding claims, wherein the 3D-printed controller elements (6) and/or the lid (3) are produced by means of laser sintering.

8. Lid configuration (1) according to any one of the preceding claims, wherein the lid (3) is a 3D-printed lid (3).

9. Bioreactor (2) comprising:
- a vessel (4), having a volume of less than 3 liters, the vessel (4) during use containing a bioreactor fluid (7),
- a lid configuration (1) according to any one of the preceding claims, connected to the vessel (4) of the bioreactor (2).

10. Bioreactor (2) according to claim 9, wherein the vessel (4) is made of plastic.

11. Bioreactor (2) according to any one of the claims 9-10, wherein the vessel (4) is a 3D-printed vessel (4).

12. Bioreactor configuration kit, comprising:
- a vessel (4), having a volume of less than 3 liters, the vessel (4) during use containing a bioreactor fluid (7),
- a lid (3) configured for connection with the vessel (4) of the bioreactor (2),
- one or more 3D-printed controller (6) elements, to be connected to the lid (3), for, during use, controlling one or more parameters of the bioreactor (2), in particular the bioreactor fluid (7), the 3D-printed controller (6) elements comprising one or more 3D-printed inlets (11) or outlets (12) comprising 3D-printed coaxial inlet (11) and/or outlet (12) tubes extending into the vessel.

13. Use of a bioreactor (8) according to one of the claims 9 - 11 or a bioreactor configuration kit according to claim 12 for growing organisms.

14. Method for producing a lid configuration (1) according to any one of the claims 1-8, comprising the steps of:
- providing a lid (3) configured for connection with the vessel (4) of the bioreactor (2), the vessel (4) during use containing a bioreactor fluid (7),
- 3D-printing one or more controller (6) elements for, during use, controlling one or more parameters of the bioreactor (2), in particular the bioreactor fluid (7), the 3D-printed controller (6) elements comprising one or more 3D-printed inlets (11) or outlets (12) comprising 3D-printed coaxial inlet (11) and/or outlet (12) tubes extending into the vessel and
- connecting the one or more controller (6) elements to the lid (3).

15. Method according to claim 14, further comprising the steps of:
- determining the process or experiment to be carried out with the bioreactor (2),
- determining the 3D-printed controller (6) elements, in particular the 3D-printed inlets (11) or outlets (12) required for carrying out the experiment or process, and
- 3D-printing only those controller (6) elements required for carrying out the experiment or process.

16. Method according to claim 15, wherein, prior to determining the 3D-printed controller (6) elements required for carrying out the experiment or process, a design is made of the bioreactor (2) for carrying out the process or experiment, and the 3D-printed controller (6) elements required for carrying out the experiment or process are determined from the design.

17. Method according to claim 14, further comprising the steps of:
- determining the process or experiment to be carried out with the bioreactor (2),
- determining the 3D-printed controller (6) elements required for carrying out the experiment or process, and
- selecting the controller (6) elements required for carrying out the experiment or process from the bioreactor configuration kit of claim 12.

## Patentansprüche

1. Deckelkonfiguration (1) für einen Bioreaktor (2), umfassend:
- einen Deckel (3), der zum Verbinden mit einem Gefäß (4) des Bioreaktors (2) konfiguriert ist, wobei das Gefäß (4) während des Gebrauchs ein Bioreaktorfluid (7) enthält, wobei der Deckel (3) mit einem oder mehreren 3D-gedruckten Elementen einer Steuerung (6) zum Steuern eines oder mehrerer Parameter des Bioreaktors (2), insbesondere des Bioreaktorfluids (7), während des Gebrauchs versehen ist, **dadurch gekennzeichnet, dass** das Gefäß ein Volumen von weniger als 3 Litern hat, wobei das eine oder die mehreren 3D-gedruckten Elemente der Steuerung (6) einen oder mehrere 3D-gedruckte Einlässe (11) oder Auslässe (12) umfassen umfassend 3D-gedruckten koaxialen Einlass- (11) und/oder Auslassrohren (12), die sich in das Gefäß hinein erstrecken.

2. Deckelkonfiguration (1) nach Anspruch 1, wobei das eine oder die mehreren 3D-gedruckten Elemente der Steuerung (6) ein 3D-gedrucktes Rührelement (8) umfassen.

3. Deckelkonfiguration (1) nach Anspruch 2, wobei das Rührelement (8) ein 3D-gedrucktes Rührwerk, wie etwa eine Schraube (9), umfasst.

4. Deckelkonfiguration (1) nach Anspruch 2 oder 3, wobei das Rührelement (8) eine 3D-gedruckte Welle (10) umfasst.

5. Deckelkonfiguration (1) nach einem der vorhergehenden Ansprüche, wobei die 3D-gedruckten Elemente der Steuerung (6) und/oder der Deckel (3) aus einem biologisch abbaubaren Material hergestellt sind, wie etwa einem biologisch abbaubaren Kunststoff.

6. Deckelkonfiguration (1) nach einem der vorhergehenden Ansprüche, wobei die 3D-gedruckten Elemente der Steuerung (6) und/oder der Deckel (3) aus einem biokompatiblen Material hergestellt sind.

7. Deckelkonfiguration (1) nach einem der vorhergehenden Ansprüche, wobei die 3D-gedruckten Elemente der Steuerung (6) und/oder der Deckel (3) mittels Laser-Sintern hergestellt werden.

8. Deckelkonfiguration (1) nach einem der vorhergehenden Ansprüche, wobei der Deckel (3) ein 3D-gedruckter Deckel (3) ist.

9. Bioreaktor (2), umfassend:
- ein Gefäß (4), mit einem Volumen von weniger als 3 Litern, wobei das Gefäß (4) während des Gebrauchs ein Bioreaktorfluid (7) enthält,
- eine Deckelkonfiguration (1) nach einem der vorhergehenden Ansprüche, die mit dem Gefäß (4) des Bioreaktors (2) verbunden ist.

10. Bioreaktor (2) nach Anspruch 9, wobei das Gefäß (4) aus Kunststoff hergestellt ist.

11. Bioreaktor (2) nach einem der Ansprüche 9 bis 10, wobei das Gefäß (4) ein 3D-gedrucktes Gefäß (4) ist.

12. Bioreaktorkonfigurationskit, umfassend:
- ein Gefäß (4), mit einem Volumen von weniger als 3 Litern, wobei das Gefäß (4) während des Gebrauchs ein Bioreaktorfluid (7) enthält,
- einen Deckel (3), der zur Verbindung mit dem Gefäß (4) des Bioreaktors (2) konfiguriert ist,
- ein oder mehrere 3D-gedruckte Elemente der Steuerung (6), die mit dem Deckel (3) zu verbinden sind, um während des Gebrauchs einen oder mehrere Parameter des Bioreaktors (2), insbesondere des Bioreaktorfluids (7), zu steuern, wobei die 3D-gedruckten Elemente der Steuerung (6) einen oder mehrere 3D-gedruckte Einlässe (11) oder Auslässe (12) umfassen umfassend 3D-gedruckten koaxialen Einlass- (11) und/oder Auslassrohren (12), die sich in das Gefäß hinein erstrecken.

13. Verwendung eines Bioreaktors (8) nach einem der Ansprüche 9 bis 11 oder eines Bioreaktorkonfigurationskits nach Anspruch 12 für wachsende Organismen.

14. Verfahren zum Herstellen einer Deckelkonfiguration (1) nach einem der Ansprüche 1 bis 8, die folgenden Schritte umfassend:
- Bereitstellen eines Deckels (3), der zur Verbindung mit dem Gefäß (4) des Bioreaktors (2) konfiguriert ist, wobei das Gefäß (4) während des Gebrauchs ein Bioreaktorfluid (7) enthält,
- 3D-Drucken eines oder mehrerer Elemente der Steuerung (6) zum Steuern eines oder mehrerer Parameter des Bioreaktors (2), insbesondere des Bioreaktorfluids (7), während des Gebrauchs, wobei die 3D-gedruckten Elemente der Steuerung (6) einen oder mehrere 3D-gedruckte Einlässe (11) oder Auslässe (12) umfassen umfassend 3D-gedruckten koaxialen Einlass- (11) und/oder Auslassrohren (12), die sich in das Gefäß hinein erstrecken, und
- Verbinden des einen oder der mehreren Elemente der Steuerung (6) mit dem Deckel (3).

15. Verfahren nach Anspruch 14, ferner die folgenden Schritte umfassend:
- Bestimmen des Prozesses oder Experiments, der/das mit dem Bioreaktor (2) durchgeführt werden soll,
- Bestimmen der 3D-gedruckten Elemente der Steuerung (6), insbesondere der 3D-gedruckten Einlässe (11) oder Auslässe (12), die zur Durchführung des Experiments oder Prozesses erforderlich sind, und
- 3D-Drucken nur der Elemente der Steuerung (6), die zur Durchführung des Experiments oder Prozesses erforderlich sind.

16. Verfahren nach Anspruch 15, wobei vor dem Bestimmen der 3D-gedruckten Elemente der Steuerung (6), die zur Durchführung des Experiments oder Prozesses erforderlich sind, ein Entwurf des Bioreaktors (2) zur Durchführung des Prozesses oder Experiments hergestellt wird und die 3D-gedruckten Elemente der Steuerung (6), die zur Durchführung des Experiments oder Prozesses erforderlich sind, anhand des Entwurfs bestimmt werden.

17. Verfahren nach Anspruch 14, ferner die folgenden Schritte umfassend:
- Bestimmen des Prozesses oder Experiments, der/das mit dem Bioreaktor (2) durchgeführt werden soll,
- Bestimmen der 3D-gedruckten Elemente der Steuerung (6), die zur Durchführung des Experiments oder Prozesses erforderlich sind, und
- Auswählen der Elemente der Steuerung (6), die zur Durchführung des Experiments oder Prozesses erforderlich sind, aus dem Bioreaktorkonfigurationskit nach Anspruch 12.

## Revendications

1. Configuration de couvercle (1) de bioréacteur (2), comprenant :
- un couvercle (3) conçu pour être relié à un récipient (4) du bioréacteur (2), le récipient (4) pendant l'utilisation contenant un fluide de bioréacteur (7), le couvercle (3) étant pourvu d'un ou plusieurs éléments de dispositif de commande (6) imprimés en 3D pour, pendant l'utilisation, commander un ou plusieurs paramètres du bioréacteur (2), en particulier le fluide de bioréacteur (7), **caractérisé en ce que** le récipient a un volume inférieur à 3 litres, dans laquelle les un ou plusieurs éléments de dispositif de commande (6) imprimés en 3D comprennent une ou plusieurs entrées (11) ou sorties (12) imprimées en 3D comprenant des tubes coaxiaux d'entrée (11) et/ou de sortie (12) imprimés en 3D s'étendant dans le récipient.

2. Configuration de couvercle (1) selon la revendication 1, dans laquelle les un ou plusieurs éléments de dispositif de commande (6) imprimés en 3D comprennent un élément d'agitation (8) imprimé en 3D.

3. Configuration de couvercle (1) selon la revendication 2, dans laquelle l'élément d'agitation (8) comprend un impulseur imprimé en 3D, tel qu'une vis (9).

4. Configuration de couvercle (1) selon la revendication 2 ou 3, dans laquelle l'élément d'agitation (8) comprend un arbre (10) imprimé en 3D.

5. Configuration de couvercle (1) selon l'une quelconque des revendications précédentes, dans laquelle les éléments de dispositif de commande (6) imprimés en 3D et/ou le couvercle (3) sont constitués d'un matériau biodégradable, tel qu'un plastique biodégradable.

6. Configuration de couvercle (1) selon l'une quelconque des revendications précédentes, dans laquelle les éléments de dispositif de commande (6) imprimés en 3D et/ou le couvercle (3) sont constitués d'un matériau biocompatible.

7. Configuration de couvercle (1) selon l'une quelconque des revendications précédentes, dans laquelle les éléments de dispositif de commande (6) imprimés en 3D et/ou le couvercle (3) sont produits au moyen d'un frittage laser.

8. Configuration de couvercle (1) selon l'une quelconque des revendications précédentes, dans laquelle le couvercle (3) est un couvercle (3) imprimé en 3D.

9. Bioréacteur (2) comprenant :
- un récipient (4), ayant un volume inférieur à 3 litres, le récipient (4) pendant l'utilisation contenant un fluide de bioréacteur (7),
- une configuration de couvercle (1) selon l'une quelconque des revendications précédentes, reliée au récipient (4) du bioréacteur (2).

10. Bioréacteur (2) selon la revendication 9, dans lequel le récipient (4) est constitué de plastique.

11. Bioréacteur (2) selon l'une quelconque des revendications 9 à 10, dans lequel le récipient (4) est un récipient (4) imprimé en 3D.

12. Kit de configuration de bioréacteur, comprenant :
- un récipient (4), ayant un volume inférieur à 3 litres, le récipient (4) pendant l'utilisation contenant un fluide de bioréacteur (7),
- un couvercle (3) conçu pour être relié au récipient (4) du bioréacteur (2),
- un ou plusieurs éléments de dispositif de commande (6) imprimés en 3D, à relier au couvercle (3), pour, pendant l'utilisation, commander un ou plusieurs paramètres du bioréacteur (2), en particulier le fluide de bioréacteur (7), les éléments de dispositif de commande (6) imprimés en 3D comprenant une ou plusieurs entrées (11) ou sorties (12) imprimées en 3D comprenant des tubes coaxiaux d'entrée (11) et/ou de sortie (12) imprimés en 3D s'étendant dans le récipient.

13. Utilisation d'un bioréacteur (8) selon l'une des revendications 9 à 11 ou d'un kit de configuration de bioréacteur selon la revendication 12 pour des organismes en croissance.

14. Procédé de production d'une configuration de couvercle (1) selon l'une quelconque des revendications 1 à 8, comprenant les étapes :
- de fourniture d'un couvercle (3) conçu pour être relié au récipient (4) du bioréacteur (2), le récipient (4) pendant l'utilisation contenant un fluide de bioréacteur (7),
- d'impression 3D d'un ou plusieurs éléments de dispositif de commande (6) pour, pendant l'utilisation, commander un ou plusieurs paramètres du bioréacteur (2), en particulier le fluide de bioréacteur (7), les éléments de dispositif de commande (6) imprimés en 3D comprenant une ou plusieurs entrées (11) ou sorties (12) imprimées en 3D comprenant des tubes coaxiaux d'entrée (11) et/ou de sortie (12) imprimés en 3D s'étendant dans le récipient et
- de liaison des un ou plusieurs éléments de dispositif de commande (6) avec le couvercle (3).

15. Procédé selon la revendication 14, comprenant en outre les étapes :
- de détermination de la procédure ou de l'expérience à réaliser avec le bioréacteur (2),
- de détermination des éléments de dispositif de commande (6) imprimés en 3D, en particulier les entrées (11) ou les sorties (12) imprimées en 3D nécessaires à la réalisation de l'expérience ou de la procédure, et
- d'impression en 3D uniquement des éléments de dispositif de commande (6) nécessaires à la réalisation de l'expérience de la procédure.

16. Procédé selon la revendication 15, dans lequel, avant de déterminer les éléments de dispositif de commande (6) imprimés en 3D nécessaires à la réalisation de l'expérience ou de la procédure, un dessin du bioréacteur (2) est fait pour réaliser la procédure ou l'expérience, et les éléments de dispositif de commande (6) imprimés en 3D nécessaires à la réalisation de l'expérience ou de la procédure sont déterminés à partir du dessin.

17. Procédé selon la revendication 14, comprenant en outre les étapes :
- de détermination de la procédure ou de l'expérience à réaliser avec le bioréacteur (2),
- de détermination des éléments de dispositif de commande (6) imprimés en 3D nécessaires à la réalisation de l'expérience ou de la procédure, et
- de sélection des éléments de dispositif de commande (6) nécessaires à la réalisation de l'expérience ou de la procédure à partir du kit de configuration de bioréacteur selon la revendication 12.
